# EUROPÄISCHE PATENTSCHRIFT

(11) **EP 0 663 404 B1**
(45) Veröffentlichungstag und Bekanntmachung des Hinweises auf die Patenterteilung: **10.04.2002**
(21) Anmeldenummer: 95100067.8
(22) Anmeldetag: 04.01.1995
(51) Int. Cl.: C07K 14/255, C07K 16/12, A61K 38/10, A61K 39/112

(54) **Antikörper für den Nachweis von Salmonellen**
Antibodies for the detection of Salmonella
Des anticorps pour la détection de la Salmonelle

(30) Priorität: 14.01.1994 DE 4400990
(43) Veröffentlichungstag der Anmeldung: 19.07.1995
(73) Patentinhaber: MERCK PATENT GmbH, 64293 Darmstadt (DE)
(72) Erfinder: Schubert, Dr. Peter, D-64285 Darmstadt (DE); Neumann, Dr. Siegfried, D-64342 Seeheim-Jugenheim (DE); Burger, Dr. Christa, D-64289 Darmstadt (DE); Linxweiler, Dr. Winfried, D-64823 Gross-Umstadt (DE); Bubert, Dr. Andreas, D-97218 Gerbrunn (DE); Goebel, Prof. Dr. Werner, D-97209 Veitshöchheim (DE); Mollenkopf, Hans-Joachim, D-97236 Randersacker (DE)

(56) Entgegenhaltungen:
- WO-A-92/17785

## Beschreibung

Die Erfindung betrifft Mittel und Verfahren zum immunologischen Nachweis von Bakterien der Gattung Salmonella, sowie ausgewählte Peptide aus dem äußeren Membranprotein PagC.

Die Erreger der Gattung *Salmonella* sind gram-negative, meist begeißelte Stäbchen, die ohne große Ansprüche an das Nährmedium sowohl aerob als auch fakultativ-anaerob wachsen können. Von großer epidemiologischer Bedeutung ist die hohe Umweltresistenz dieser weit verbreiteten Bakterien. So konnten Überlebenszeiten von mehreren Wochen bis Monaten, auch bei niedrigen Temperaturen, z. B. in Lebensmitteln, Zimmerstaub, Leitungswasser, stehenden Gewässern und Gartenerde ermittelt werden. Grundsätzlich werden zwei verschiedene von Salmonellen verursachte Krankheitsformen unterschieden:
1. Typhoide Allgemeininfektion (Typhus, Paratyphus), verursacht von *Salmonella typhi* und *S. paratyphi*. Die Übertragung erfolgt in der Regel fäkal-oral oder über kontaminierte Lebensmittel.
2. Akute Gastroenteritis (Salmonellosen), verursacht von über 200 verschiedenen *Salmonella*-Serotypen. Die Übertragung erfolgt im wesentlichen über kontaminierte Lebensmittel.

Die Schwere einer Salmonellose, die sich nach oraler Aufnahme der Bakterien nach ca. 24 Stunden hauptsächlich durch Erbrechen und Durchfall äußert, ist vom Serotyp und von der Infektionsdosis abhängig. Daneben spielt auch das Lebensalter und der Immunstatus eine Rolle. Innerhalb der Familie der Enterobacteriaceae sind Salmonellen neben *E. coli* und *Shigella-*Arten die häufigsten Verursacher einer Lebensmittelvergiftung. Dabei wurde in den letzten Jahren ein sprunghafter Anstieg von Salmonellosen verzeichnet. Zwischen 1985 und 1991 hat sich in Deutschland die Zahl der gemeldeten Fälle von 30000 auf 114000 nahezu vervierfacht. Als Ursache wird zum großen Teil die Massentierhaltung, vor allem der der Hühner, und eine unzureichende Hygiene bei der Verarbeitung von Tierprodukten, sowie ein mangelndes Hygienebewußtsein in Groß- und Haushaltsküchen angesehen. Die Übertragung erfolgt hauptsächlich durch Fleischwaren, Eier, Milchprodukte und Backwaren.

Für den Bereich der Lebensmittelkontrolle, aber auch der Medizin, besteht daher ein steigender Bedarf an schnellen und sicheren Verfahren zum Nachweis von Salmonellen. Behördlich vorgeschriebene Nachweisverfahren beruhen auf Arbeitsschritten, bei denen die Bakterien auf drei verschiedenen Nährböden kultiviert werden: 1. Nichtselektive Anreicherung. 2. Selektivanreicherung. 3. Wachstum auf festen Selektivnährböden mit anschließender serologischer Typisierung. Insgesamt dauert dieses Verfahren 4-5 Tage.

Voraussetzung für eine schnelle und gezielte Behandlung von Erkrankten und für die rasche Eindämmung des Infektionsherdes sind schnelle und spezifische Verfahren für den Nachweis von Salmonellen. Gesetzliche Regelungen verlangen für den Nachweis von Salmonellen, daß die verwendeten Methoden für diese Bakterien, unabhängig von Pathogenität und Virulenz, sensitiv sein müssen. Zur Verkürzung der langen Nachweiszeiten des offiziellen Verfahrens kommen in erster Linie molekulargenetische und immunologische Methoden in Betracht.

Die Verwendung von Nukleinsäure-Sonden ist im allgemeinen nicht so empfindlich, daß auf eine Vorkultur verzichtet werden kann. Aus diesem Grunde ist das Kolonie-Hybridisierungsverfahren eine gebräuchliche, aber arbeitsintensive Verfahrensvariante. Nachweisverfahren, die auf der Amplifizierung von Nukleinsäure-Bereichen beruhen, z.B. die Polymerase-Kettenreaktion (polymerase chain reaction) verbessern im allgemeinen die Nachweisgrenze, könnten aber durch verschiedene z.B. in Lebensmitteln vorhandene Substanzen inhibiert werden (Wernars et al. 1991. J. Appl. Bacteriol. 70: 121-126).

Bewährt haben sich in der Routine-Diagnostik immunologische Nachweismethoden, jedoch sind in der Literatur bislang wenige Antikörper beschrieben worden, die zum Nachweis von Salmonellen befähigt sind. Die klassischen Antigene zur Entwicklung von Salmonellen-spezifischen Antikörpern basieren entweder auf somatischen (O-Antigene) Faktoren, z.B. einem spezifischen Teil der bakteriellen Lipopolysaccharidschicht (EP 0 494 085) oder auf Flagellen (H)-Antigene (WO 86/00-993, WO 86/04-352 und WO 89/01-162). Darüber hinaus finden bestimmte Oberflächen- (Vi)-Antigene Anwendung. Hierbei handelt es sich um Rezeptoren für Bakteriophagen (Ackerman, H.-W., Dubow, M.S. - Viruses of Prokaryotes. 2 volumes. CRC Press, Boca Raton, 1987).

In dem WHITE-KAUFMANN-LE MINOR Schema zur Identifizierung und Klassifizierung von Salmonellen werden alle drei genannten Antigenfaktoren (O, H, Vi) berücksichtigt. Bei derzeit über 2200 verschiedenen Serotypen ergeben sich hieraus zwangsläufig Unzulänglichkeiten von Screening-Tests mit Antikörpern, die gegen diese Antigenfaktoren gerichtet sind. Zur Minimierung der Nachweisprobleme werden häufig gegen verschiedene dieser Antigenfaktoren gerichtete Antikörpergemische eingesetzt. Diese Vorgehensweise ist jedoch sehr zeit- und kostenintensiv.

In WO 92/17 785 wird die Verwendung von Antikörpern, die gegen PagC gerichtet sind, für den immunologischen Nachweis von Salmonellen vorgeschlagen. Dieses Antigen, PagC aus *Salmonella* mit einem Molekulargewicht von ca. 18 Kilodalton stellt ein Protein dar, das in der äußeren Membran dieser Bakterien lokalisiert ist. Salmonellen mit einer PagC-Mutation sind avirulent, da sie in Wirtszellen nicht überlebensfähig sind. Das für PagC kodierende Gen konnte auch in anderen *Salmonella*-Spezies detektiert werden und ist offenbar in allen Salmonellen vorhanden (Pulkkinen and Miller 1991. J. Bacteriol. 173: 86-93). Die Expression von PagC ist nicht konstitutiv, sondern steht unter der Kontrolle des sogenannten Zwei-Komponenten-System PhoP/PhoQ. PhoQ stellt dabei ein Sensorprotein dar, das auf äußere Umwelteinflüsse reagiert und diese Information auf das Transmitter-Protein PhoP überträgt. Das aktivierte PhoP wiederum ist in der Lage, die Transkription des *pag*C-Gens zu stimulieren. Die Expression von PagC kann z.B. durch pH-Absenkung des Mediums, dessen Phosphat-Limitation oder auch durch stationäre Wachstumsphase verstärkt werden.

Die Expression von PagC oder ähnlichen Proteinen ist aber nicht auf *Salmonella* beschränkt. PagC-homologe Proteine konnten auch in anderen Enterobacteriaceae, (OmpX aus *Enterobacter cloacae,* Ail aus *Yersinia enterocolitica,* Lom aus Lambda-Phagen tragende *E. coli*) identifiziert werden. Aus diesem Grund sind polyklonale Antikörper, die durch Immunisierung gegen das gesamte PagC erhalten wurden, nicht geeignet zum spezifischen Nachweis von Salmonellen. In WO 92/17 785 wird weiterhin die Verwendung des Peptides Arg Gly Val Asn Val Lys Arg Tyr Glu Asp Asp Ser Phe (RGVNVKYRYEDDSF; Seq.I.D.No: 8) aus PagC als Immunogen für die Herstellung von polyklonalen Antikörpern vorgeschlagen. Die resultierenden Antikörper sind aber wegen der ausgeprägten Sequenzhomologie zu den PagC-ähnlichen Proteinen aus anderen Enterobakterien zum spezifischen Nachweis von Salmonellen ebenfalls ungeeignet.

Aufgabe der vorliegenden Erfindung ist es, verbesserte Mittel und Methoden für den immunologischen Nachweis von Bakterien der Gattung Salmonella bereitzustellen. Insbesondere werden erfindungsgemäß Peptide zur gezielten Erzeugung von spezifischen Antikörpern, die für den immunologischen Nachweis von Salmonellen geeignet sind, bereitgestellt.

Gegenstand der Erfindung sind Peptide, ausgewählt aus dem Protein PagC, mit einer Sequenz nach einer der Formeln la bis le (Seq.I.D.No: 1 bis Seq.I.D.No: 5).

Glu Pro Glu Gly Ile His Tyr His Asp Lys Phe Glu Val Ia
(Seq.I.D.No: 1)

Ala Thr Phe Lys Glu His Ser Thr Gln Asp Gly Asp Ser Ib
(Seq.I.D.No: 2)

Gly Asp Arg Ala Ser Gly Ser Val Glu Pro Glu Gly Ic
(Seq.I.D.No: 3)

Gln Ser Lys Val Gln Asp Phe Lys Asn Ile Arg Id
(Seq.I.D.No: 4)

Glu His Ser Thr Gln Asp Gly Asp Ser Phe Ser Asn Lys Ie
(Seq.I.D.No: 5)

Gegenstand der Erfindung ist auch die Verwendung eines der genannten Peptide mit einer Sequenz nach einer der Formeln la bis le (Seq.I.D.No: 1 bis Seq.I.D.No: 5) für die Herstellung von immunogenen Konjugaten.

Gegenstand der Erfindung ist auch ein Antikörper, der spezifisch gegen ein Epitop gerichtet ist, das von einem Peptid nach einer der Formeln la - le (Seq.I.D.No: 1 - Seq.I.D.No: 5), gebildet wird.

Gegenstand der Erfindung ist weiterhin die Verwendung eines Antikörpers, der gegen eines der Epitope mit einer Aminosäuresequenz nach einer der Formeln la - le (Seq.I.D.No: 1 - Seq.I.D.No: 5) gerichtet ist, für den Nachweis von Bakterien der Gattung Salmonella.

Gegenstand der Erfindung sind auch Verfahren zum Nachweis von Bakterien der Gattung Salmonella mittels eines Antikörpers, der gegen eines der Epitope mit einer Aminosäuresequenz nach einer der Formeln la - le (Seq.I.D.No: 1 - Seq.I.D.No: 5) gerichtet ist.

Gegenstand der Erfindung sind ferner Testzusammenstellungen zum immunologischen Nachweis von Bakterien der Gattung Salmonella, in denen ein Antikörper gegen ein Epitop nach einer der Formeln la - le (Seq.I.D.No: 1 - Seq.I.D.No: 5) enthalten ist.

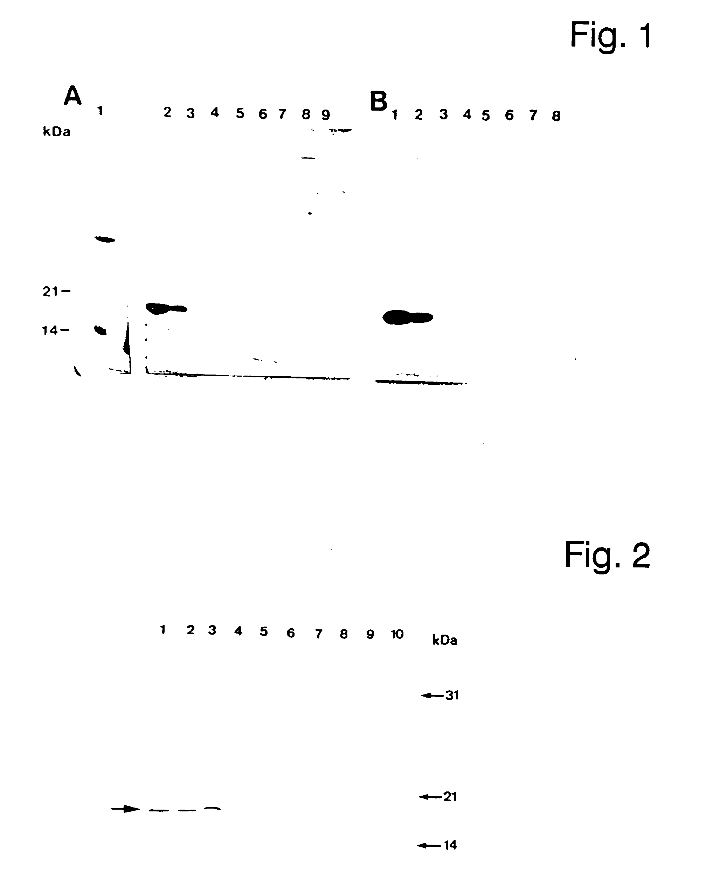

Die Abbildungen 1 bis 4 zeigen die Ergebnisse von Immunoblot-Versuchen; die experimentellen Einzelheiten finden sich in den Beispielen.

Die Erfindung wird im folgenden näher beschrieben. Dabei wird in der Regel auf Einzelheiten von biochemischen, immunologischen und molekularbiologischen Verfahren, die dem Fachmann bekannt sind, und deren Einzelheiten in der Literatur beschrieben sind, nicht näher eingegangen. Bei diesen Verfahren kann man auch von an sich bekannten, hier nicht näher beschriebenen Varianten Gebrauch machen.

Die erfindungsgemäßen Peptide nach den Formeln la - le (Seq.I.D.No: 1 - Seq.I.D.No: 5) können als immunogene Peptide zur Erzeugung von Antikörpern dienen und ermöglichen somit einen spezifischen immunologischen Nachweis von Bakterien der Gattung Salmonella.

Dem Fachmann ist bekannt, daß häufig der Austausch von einer oder wenigen Aminosäuren in einem Peptid dessen biologische Eigenschaften nicht verändert. Deswegen umfassen die erfindungsgemäßen Peptidsequenzen auch solche, die durch Aminosäureaustausch aus den Sequenzen la - le (Seq.I.D.No: 1 - Seq.I.D.No: 5) abgeleitet sind, und die biologisch dieselbe Wirkung wie die jeweiligen Peptide mit der Ursprungssequenz zeigen. Hinweise für die Möglichkeit derartiger Aminosäureaustausche entnimmt der Fachmann beispielsweise der Zusammenstellung von Dayhoff, M.O. in Atlas of Protein Sequence and Structure, Vol. **5**, Seite 98 (1972). In ähnlicher Weise hat häufig das Entfernen von einer oder wenigen Aminosäuren an einem oder beiden Enden des Peptides keinen oder geringen Einfluß auf die Immunigenität der resultierenden immunogenen Konjugate oder auf die Spezifität der resultierenden Antikörper. Gleiches gilt auch, wenn eine oder wenige Aminosäuren an einem oder beiden Enden des Peptids hinzugefügt werden. Deswegen werden diese wirkungsgleichen Varianten der erfindungsgemäßen Peptide und die mit ihrer Hilfe erzeugten Antikörper von der Erfindung mit umfaßt.

Basierend auf der Sequenz der Aminosäuren können die Peptide nach dem Fachmann bekannten Verfahren, beispielsweise dem t_{boc}- oder dem f_{moc}-Verfahren (tert.butyloxycarbonyl-, bzw. 9-fluorenylmethyloxycarbonyl-), synthetisiert werden. Einzelheiten dieser Verfahren sind beispielsweise in J.Am.Chem.Soc.**85**, 2149-2154 (1963) und in Synthetic Polypeptides as Antigens (van Regenmortel et al. (eds.) Elsevier 1988 (Band 19 der Buchreihe Laboratory Techniques in Biochemistry and Molecular Biology) beschrieben. Bevorzugt wird das f_{moc}-Verfahren, insbesondere mechanisierte Verfahrensvarianten. Einzelheiten des Verfahrens, sowie geeignete Aminosäureschutzgruppen sind dem Fachmann bekannt.

Peptide sind im allgemeinen nicht geeignet, Antikörper zu erzeugen. Werden Peptide jedoch an hochmolekulare Trägersubstanzen gekoppelt, so entstehen immunogene Konjugate. Die erfindungsgemäßen Peptide lassen sich mit bekannten Trägersubstanzen konjugieren. Dazu gehören Polyäthylenglykole, Serumalbumine, KLH (Keyhole Limpet Hemocyanin; Hämocyanin von Napfschnecken), Ovalbumin, Glucosedehydrogenase aus Bacillus megatherium und PPD (purified protein derivative of tuberculin). Bevorzugte Trägersubstanzen sind KLH und Glucosedehydrogenase aus B. megatherium.

Bei der Synthese der Konjugate werden zusätzlich häufig Brückenverbindungen (linker) eingesetzt. Dies sind niedermolekulare organische Verbindungen mit mindestens zwei verknüpfbaren funktionellen Gruppen. Geeignete Verbindungen sind dem Fachmann bekannt; dazu gehören beispielsweise: 1,2-Diaminoethan, Bemsteinsäure, β-Alanin, 1,6-Diaminohexan, 6-Aminocapronsäure, Adipinsäure, Cystein. Bevorzugt wird Cystein als Linker eingesetzt, wobei dieser Aminosäurerest bereits bei der Synthese des Peptides eingebaut wird. Zur Herstellung der Bindungen zwischen Peptid und Trägersubstanz wird bevorzugt m-Maleimidobenzoyl-N-hydroxysuccinimidester (MBS) eingesetzt.

Die genannten immunogenen Konjugate dienen dazu, nach bekannten Verfahren in Versuchstieren Antikörper zu erzeugen. Üblicherweise werden für diesen Zweck Säugetiere, beispielsweise Schaf, Ziege, Kaninchen oder Mäuse benutzt. Kaninchen sind für die Erzeugung polyklonaler Antikörper bevorzugt. Es ist aber auch möglich, mit Hilfe der erfindungsgemäßen immunogenen Konjugate monoklonale Antikörper herzustellen.

Einzelheiten der immunologischen Verfahren sind dem Fachmann bekannt. Hinweise zur Durchführung dieser Verfahren sind außerdem vielfältig in der Literatur anzutreffen; beispielsweise seien genannt:
* Antibodies, E. Harlow und D. Lane, Cold Spring Harbor (1988)
* Woodard, L.F. und Jasman, R.L. (1985) Vaccine **3**, 137-144
* Woodard, L.F. (1989) Laboratory Animal Sci **39,** 222-225
* Handbook of Experimental Immunology (1986) Weir, D.M. et al. eds.; Blackwell Scientific Publications, Oxford, GB
Zu diesen Verfahren gehören beispielsweise die Konjugations- und Immunisierungsverfahren, sowie die Herstellung und Aufreinigung von Antikörpern und auch immunologische Nachweisverfahren. Zu den immunologischen Nachweisverfahren, bei denen erfindungsgemäße Antikörper benutzt werden können, gehören bevorzugt Agglutinationsverfahren, immunometrische Nachweisverfahren, die Immuno-Blot-Verfahren und insbesondere die sandwich-(ELISA)-Verfahren.

Der Begriff Antikörper umfaßt erfindungsgemäß sowohl Immunglobuline als auch Antiseren. Es ist dem Fachmann weiterhin bekannt, daß bei derartigen Verfahren statt der Benutzung eines einzigen Antikörpers, der gegen ein einzelnes Epitop gerichtet ist, eine Mischung von verschiedenen Antikörpern mit verschiedener Spezifität Vorteile, insbesondere bezüglich der Nachweisempfindlichkeit, erbringt. Dies trifft im besonderen für monoklonale Antikörper, aber auch für andere Antikörper zu, die gegen jeweils ein Epitop gerichtet sind. Entsprechend kann es vorteilhaft sein, mehrere Antikörper, die gegen verschiedene Peptidstrukturen nach den Formeln la - le (Seq.I.D.No: 1 - Seq.I.D.No: 5) gerichtet sind, für die erfindungsgemäße Verwendung und/oder die erfindungsgemäßen Verfahren zu kombinieren.

Einzelheiten der Herstellung der erfindungsgemäßen Peptide, sowie ihrer Verwendung sind aus den folgenden Beispielen ersichtlich. Weitere methodische Einzelheiten entnimmt der Fachmann der zitierten Literatur. Die Beispiele sollen den Gegenstand der Erfindung illustrieren und stellen keine Einschränkung der Erfindung dar.

### Beispiele:

### Beispiel 1: Chemische Synthese des Peptides CysGluHisSerThrGln AspGlyAspSerPheSerAsnLys (Seq.I.D.No: 6)

Für die chemische Synthese des Peptides CysGluHisSerThrGlnAspGlyAspSerPheSerAsnLys (Seq.I.D.No: 6) wird das f_{moc}-Verfahren (9-Fluoroenylmethyloxycarbonyl-Schutzgruppe) verwendet. Dieses Peptid entspricht einem Peptid der Formel le (Seq.I.D.No: 5) mit einem zusätzlichen N-terminalen Cystein-Rest als Linker. Für die Synthese wird ein Peptid-Synthesizer der Fa. Applied Biosystems benutzt; die Prozeßparameter sind in der Gerätedokumentation enthalten.
Ein polymerer Träger mit 4-(2',4'-Dimethoxyphenylaminomethyl)-phenoxygruppen dient als Festphase. Die Aminosäuren werden als α-N-f_{moc}-Derivate eingesetzt. Soweit die Aminosäuren reaktive Seitenketten enthalten, werden diese durch zusätzliche Schutzgruppen, die durch Trifluoroessigsäure-Hydrolyse abspaltbar sind, maskiert. Die Peptidbindungen werden durch Aktivierung der Carboxylgruppen mittels Diisopropylcarbodiimid hergestellt. Die Reihenfolge der eingesetzten Aminosäurederivate ergibt sich aus der gewünschten Sequenz. Im ersten Schritt des Synthesezyklus reagiert die Aminogruppe an der Festphase, d.h. im ersten Zyklus die Aminogruppen des 4-(2',4'-Dimethoxyphenylaminomethyl)-phenoxy-Rests des Trägers, in den folgenden Zyklen die α-Aminogruppe der zuletzt angefügten Aminosäure, die als α-N-f_{moc}-Derivat, gegebenenfalls mit geschützten Seitenketten eingesetzt wird, und die durch Diisopropylcarbodiimid aktiviert wird. Nicht umgesetzte Aminosäuren werden mit Dimethylformamid ausgewaschen. Anschließend wird die f_{moc}-Gruppe durch Behandeln mit 20% (V/V) Piperidin in Dimethylformamid abgespalten. Die übrigen Schutzgruppen bleiben bei dieser Reaktion unverändert. Mit der Entfernung der α-N-Schutzgruppe kann der nächste Reaktionszyklus beginnen. Nachdem die letzte Aminosäure entsprechend der vorgebenen Sequenz zugefügt worden ist, werden die Schutzgruppen der Seitenketten und die Bindung zum Trägerharz durch saure Hydrolyse mittels Trifluoroessigsäure gespalten. Das Peptid wird anschließend durch Hochdruckflüssigkeitschromatographie gereinigt.

Entsprechend der oben beschriebenen Vorgehensweise werden auch die übrigen Peptide mit den erfindungsgemäßen Sequenzen synthetisiert. Für Vergleichszwecke wird zusätzlich folgendes Peptid II (Seq.I.D.No: 7), dessen Sequenz ebenfalls aus der PagC-Sequenz hergeleitet ist, hergestellt:

Val Gly Tyr Glu Gly Ser Asn Ile Ser Ser Thr Lys Ile Asn II
(Seq.I.D.No: 7)

### Beispiel 2: Konjugation des Peptides CysGluHisSerThrGlnAspGlyAspSerPhe SerAsnLys (Seq.I.D.No: 6) mit Glucosedehydrogenase

### a) Derivatisierung der Glucosedehydrogenase:

30 mg Glucosedehydrogenase aus *Bacillus megaterium* (Fa. Merck, Art. Nr. 13732) werden in 4 ml Natriumphosphatpuffer (50 mM; pH 8,0) gelöst. Zu 2,4 ml dieser Lösung werden 6,78 mg N-γ-Maleimidobutyryloxysuccinimid (Fa. Calbiochem) gelöst in 50 µl Dimethylsulfoxid zugegeben und 30 Minuten bei Raumtemperatur stehen gelassen. Anschließend wird das überschüssige N-γ-Maleimidobutyryloxysuccinimid durch Gelfiltration an PD10-Säulen (Fa. Pharmacia) chromatographisch abgetrennt. Nach der Chromatographie erhält man 3,5 ml Lösung des aktivierten Trägerproteins mit einer Konzentration von 4,5 mg/ml.

### b) Kopplung mit dem Peptid:

Zu 1,1 ml der Lösung aus dem obigen Schritt werden 5,2 mg des Peptides, hergestellt nach Beispiel 1, gelöst in 1 ml Natriumphosphatpuffer (50 mM; pH 8,0) zugefügt und 3 Stunden bei Raumtemperatur stehen gelassen. Anschließend wird das nicht-gebundene Peptid durch Gelfiltration an PD10-Säulen chromatographisch abgetrennt. Nach der Chromatographie erhält man 3,5 ml Lösung des Konjugates mit einer Konzentration von 2,3 mg/ml.

Entsprechend der oben beschriebenen Vorgehensweise werden auch Konjugate mit den anderen in Beispiel 1 genannten Peptiden hergestellt.

### Beispiel 3: Erzeugung von polyklonalen Antikörpern gegen das Peptid CysGluHisSerThrGlnAspGlyAspSerPheSerAsnLys (Seq.I.D.No: 6):

Zwei Mäuse werden jeweils mit einer Emulsion aus 0,1 ml Konjugat aus Beispiel 2 und 0,1 ml Öladjuvans (MISA 50, Fa. Seppic, FR.) s.c. injiziert. Drei, fünf und sieben Wochen nach der Erstinjektion erfolgen Boosterinjektionen mit gleicher Menge. Eine Woche nach der letzten Injektion werden die Tiere getötet und ausgeblutet. Nachdem das Blut geronnen ist, wird Antiserum durch Zentrifugation gewonnen und Natriumazid bis zu einer Endkonzentration von 0,02% zugefügt. Das Antiserum wird bei -20°C eingefroren gelagert.

Entsprechend der oben beschriebenen Vorgehensweise werden auch die nach Beispiel 2 erzeugten anderen Peptid-Konjugate zur Immunisierung verwendet.

### Beispiel 4: Erzeugung von monoklonalen Antikörpern gegen das Peptid CysGluHisSerThrGlnAspGlyAspSerPheSerAsnLys (Seq.I.D.No: 6):

Zwei Mäuse werden jeweils mit einer Emulsion aus 0,1 ml Konjugat aus Beispiel 2 und 0,1 ml Öladjuvans (MISA 50, Fa. Seppic, FR.) s.c. injiziert. Zwei, vier und sechs Wochen nach der Erstinjektion erfolgen Boosterinjektionen mit gleicher Menge. Drei Tage nach der letzten Injektion werden die Tiere getötet und die Milz isoliert. die Zellen aus der Milz werden nach üblichen Verfahren isoliert und mit einer permanenten murinen Zellinie fusioniert. Aus den Fusionsprodukten werden Zellinien selektioniert, die Antikörper gegen das Peptid CysGluHisSerThrGlnAspGlyAspSerPheSerAsnLys bilden.

Entsprechend der oben beschriebenen Vorgehensweise werden auch die nach Beispiel 2 erzeugten anderen Peptid-Konjugate zur Herstellung monoklonaler Antikörper verwendet.

### Beispiel 5: Spezifischer immunologischer Nachweis von Salmonellen

### a) Vorkultur der Enterobakterien

Verschiedene zu analysierende Enterobakterien werden jeweils in 10 ml YT-Medium angeimpft und bei 37° C über Nacht inkubiert.

### b) Vorbehandlung der Salmonellen für eine SDS-Extraktion der äußeren Membranproteine

Es werden 2 ml aus jeder Kultur entnommen. Die Bakterienzellen werden abzentrifugiert (13000 UpM; 5 min), in 2 ml Tris-gepufferter Saline (TBS: 10 mM Tris-HCI pH 7,5; 0,9 % NaCl) resuspendiert und erneut abzentrifugiert. Zur Extraktion von äußeren Membranproteinen wird der Bakterien-Niederschlag anschließend in 2 ml TBS, in dem 0,025% Natriumdodecylsulfat (SDS) enthalten sind, resuspendiert und für 45 Minuten inkubiert. Nach 10 minütiger Zentrifugation werden 1,8 ml Überstand in ein neues Reaktionsgefäß überführt und die im Extrakt enthaltenen Proteine durch Zugabe von Trichloressigsäure (10% Endkonzentration) für 1,5 Stunden auf Eis ausgefällt. Nach 15 minütiger Zentrifugation wird der Niederschlag in 500 µl Aceton gewaschen, für 10 Minuten zentrifugiert und nach Entfernen des Acetons kurz luftgetrocknet. Der Niederschlag wird anschließend in Lämmli-Probenpuffer gelöst, mit 1 µl 2 N NaOH versetzt, und für 5 Minuten bei 95°C aufgekocht.

### c) Vorbehandlung der Enterobakterien für eine Zellfraktionierung

Aus der Übernachtkultur werden 0,5 ml entnommen, 1:25 mit YT-Medium verdünnt, und bis zu einer optischen Dichte, gemessen bei 600 nm, von 0,7 bei 37°C angezüchtet. Die Bakterien-Suspension wird mit 0,5 M Natriumcitrat auf einen pH von 4,8 gebracht und für 2 weitere Stunden inkubiert. Die Zellen werden nach der Methode von Neu und Heppel (1965. J. Bacteriol. 240: 3685-3692) durch zweimaliges Einfrieren und Auftauen und anschließender Ultraschallungbehandlung (4 x 30 Sekunden) aufgeschlossen. Die Gewinnung der Membranfraktion erfolgt durch Sedimentation in einer Ultrazentrifuge für 30 Minuten bei 100 000 x g. Die sedimentierten Proteine werden in Lämmli-Probenpuffer (1,52 g Tris; 20 ml Glycerol; 2g SDS; 2 ml 2-Mercaptoethanol; 1 mg Bromphenolblau; ad 100 ml mit H₂O; pH 6,8 mit HCI) und NaOH gelöst und 5 Minuten bei 95°C denaturiert. Anschließend werden die Proteine bei 150 Volt mittels einer diskontinuierlichen SDS-Polyacrylamidgelelektrophorese (kombiniertes Trenn- und Sammelgel) aufgetrennt. Zusammensetzung des Trenngels: 11,25 ml Tris-HCI pH 8,8; 5,46 ml H₂O; 12,45 ml 30% Acrylamid/0,8% Bisacrylamid; 0,3 ml 10% SDS; 0,36 ml Ammoniumperoxodisulfat (32 mg/ml); 30 µl TEMED (*N*,*N*,*N'*,*N'*-Tetramethylethylendiamin). Sammelgel: 2,5 ml Tris-HCI pH 6,8; 14,7 ml H₂O; 2 ml Acrylamid/0,8% Bisacrylamid; 0,2 ml SDS; 0,4 ml Ammoniumperoxodisulfat (32 mg/ml); 20 µl TEMED.
Die im Gel aufgetrennten Proteine werden anschließend durch ein Semi-trockenes Elektroblotting-Verfahren in einer Graphit-Kammer (Kyhse-Andersen. 1984. J. Biochem. Biophys. Methods 10: 203-209) aus dem Gel auf eine Nitrozellulose-Membran (Hybond-C® ; Fa. Amersham) überführt.

Die Nitrozellulose-Membran mit den transferierten Proteinen wird zur Blockierung freier Proteinbindungsstellen für eine Stunde in einer Lösung, bestehend aus Rinderserumalbumin (BSA; 10 g/L) in phosphatgepufferter Saline (PBS) geschwenkt. Das in Beispiel 3 gewonnene Antiserum wird in PBS/BSA (10 g/L)/Polysorbat 20 (Tween® 20; ICI America; 0,5 g/L) verdünnt (1:250, wenn nicht anders angegeben) und für eine Stunde mit der behandelten Membran bei Raumtemperatur inkubiert. Nach dreimaligem Waschen der Membran in PBS/ Tween® 20 (0,5 g/L) wird die Membran mit einem Peroxidase-markiertem anti-Maus Antikörper, der zuvor in demselben Verdünnungspuffer 1:750 verdünnt wurde, für eine Stunde bei Raumtemperatur inkubiert. Nach zweimaligem Waschen in PBS/Tween® 20 (0,5 g/L) und einmaligem Waschen in Tris-gepufferter Saline (TBS) wird die Substratlösung (4-Chloro-1-naphthol: 3 mg/ml in Methanol + 5 Volumina TBS + Wasserstoffperoxid-Endkonzentration 0,1 g/L) dazugegeben. Die Farbreaktion auf der Membran (Entstehen einer Blauschwarz-Färbung) zeigt die Reaktivität des Antiserums mit den Proteinen.

### Ergebnisse der Immunoblots

Mit den nach Beispiel 3 erzeugten Peptid-Antiseren werden Immunoblots wie oben beschrieben ausgeführt:
Abbildung 1 zeigt Immunoblots der Antiseren (1:500 verdünnt) gegen Peptid Id (Seq.I.D.No: 4; A) und le (Seq.I.D.No: 5; B) mit Proteinaufarbeitungen aus verschiedenen Enterobakterien. Beide analysierten *Salmonella*-Isolate *S. typhimurium* und *S*. *choleraesuis,* die phylogenetisch innerhalb der Gattung *Salmonella* sehr divergent sind, können spezifisch über die Reaktivität der produzierten Antikörper gegen PagC aus *Salmonella* nachgewiesen werden. Die untersuchten Enterobakteriaceae waren im einzelnen:

| Nummer | Blot A | Blot B |
|---|---|---|
| 1 | Molekulargewichtsstandard | Salmonella typhimurium |
| 2 | Salmonella typhimurium | Salmonella choleraesuis |
| 3 | Salmonella choleraesuis | Yersinia enterolytica |
| 4 | Yersinia enterolytica | Enterobacter cloacae |
| 5 | Enterobacter cloacae | Escherichia coli |
| 6 | Escherichia coli | Shigella flexneri |
| 7 | Shigella flexneri | Citrobacter diversus |
| 8 | Citrobacter diversus | Proteus mirabilis |
| 9 | Proteus mirabilis | |

Abbildung 2 zeigt die Analyse von Membranfraktionen verschiedener Enterobakterien mit einem Antiserum gegen Peptid la (Seq.I.D.No: 1; 1:200) im Immunoblot. Das PagC-Protein der drei analysierten *Salmonella*-Isolate *S*. *typhimurium* (1), *S*. *enteritidis* (2) und *S*. *typhi* (3) ist im Molekulargewichtsbereich bei 18 kDa eindeutig identifizierbar. Die übrigen untersuchten Bakterien waren: E. coli 5K (4), E coli (5), C. diversus (6), Enterobacter cloacae (7), Shigella flexneri (8), Y. enterolytica (9) und Bordetella bronchoseptica (10); in allen diesen Fällen wurde keine Reaktion beobachtet.

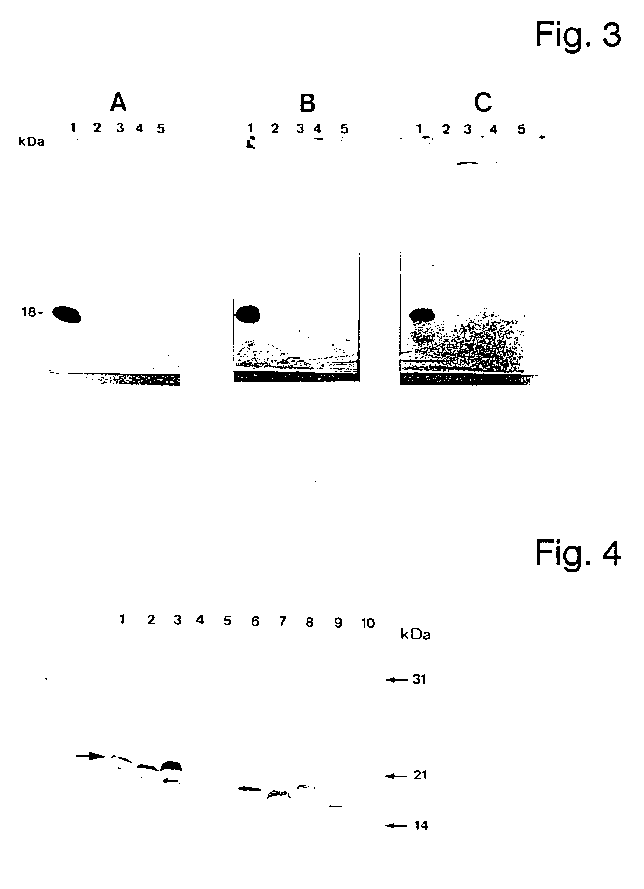

Abbildung 3 zeigt die Analyse von SDS-extrahierten Proteinen im Immunoblot (Verdünnung 1:750). Dabei wurden Antiseren gegen Peptid la (Seq.I.D.No: 1; A), Peptid Ib (Seq.I.D.No: 2; B) und Peptid Ic (Seq.I.D.No: 3; C) mit SDS-extrahierten Proteinen aus verschiedenen Enterobakterien zur Reaktion gebracht. Die untersuchten Bakterien waren im einzelnen: S. typhimurium (1), E. coli (2), C. diversus (3), Y. enterolytica (4) und Enterobacter cloacae (5).

### Vergleichsbeispiel A: Unspezifische Detektion von PagC mit polyklonalen Antikörpern gegen PagC

Antikörper gegen rekombinantes PagC Protein (Gesamtmolekül) werden auf Nitrozellulose-Membranen mit isolierten Proteinen aus verschiedenen Bakterien (siehe Beispiel 5) inkubiert. Die untersuchten Bakterien waren im einzelnen: *S*. *typhimurium* (1), *S*. *enteritidis* (2), *S*. *typhi* (3), E. coli 5K (4), E. coli (5), C. diversus (6), Enterobacter cloacae (7), Shigella flexneri (8), Y. enterolytica (9) und Bordetella bronchoseptica (10). Wie die Anfärbungen im Molekulargewichtsbereich 16-20 Kilodalton in Abbildung 4 belegen, erkennen diese Antikörper auch die PagC-homologen Proteine aus *Enterobacter cloacae* und *Yersinia enterocolitica* bzw. andere bislang nicht charakterisierte Proteine aus anderen Enterobakterien und sind deshalb für einen spezifischen immunologischen Nachweis für Salmonellen nicht geeignet.

### Vergleichsbeispiel B: Reaktion von Antikörpern gegen ein weiteres aus der PagC-Sequenz ausgewähltes Peptid

Das Antiserum gegen das Peptid II (Seq.I.D.No: 7), 
Val Gly Tyr Glu Gly Ser Asn Ile Ser Ser Thr Lys Ile Asn II
(Seq.I.D.No: 7)
 das wie die Peptide nach Formel la - le (Seq.I.D.No: 1 - Seq.I.D.No: 5) aus der PagC-Sequenz ausgewählt wurde, zeigte keine Reaktivität mit dem PagC Protein aus *Salmonella.*

## Patentansprüche

1. Peptid, ausgewählt aus dem Protein PagC, **gekennzeichnet durch** eine Sequenz nach einer der Formeln la bis le (Seq.I.D.No: 1 - Seq.I.D.No: 5).

Glu Pro Glu Gly Ile His Tyr His Asp Lys Phe Glu Val Ia
(Seq.I.D.No: 1)

Ala Thr Phe Lys Glu His Ser Thr Gln Asp Gly Asp Ser Ib
(Seq.I.D.No: 2)

Gly Asp Arg Ala Ser Gly Ser Val Glu Pro Glu Gly Ic
(Seq.I.D.No: 3)

Gln Ser Lys Val Gln Asp Phe Lys Asn Ile Arg Id
(Seq.I.D.No: 4)

Glu His Ser Thr Gln Asp Gly Asp Ser Phe Ser Asn Lys le
(Seq.I.D.No: 5)

2. Verwendung eines Peptides nach Anspruch 1 zur Herstellung von immunogenen Konjugaten.

3. Antikörper, der spezifisch gegen ein Epitop gerichtet ist, welches von einem Peptid nach Anspruch 1 gebildet wird.

4. Verwendung eines Antikörpers nach Anspruch 3 für den Nachweis von Bakterien der Gattung Salmonella.

5. Verfahren zum Nachweis von Bakterien der Gattung Salmonella mittels einer Immunreaktion, **dadurch gekennzeichnet, daß** ein Antikörper nach Anspruch 3 verwendet wird.

6. Testzusammenstellung zum Nachweis von Bakterien der Gattung Salmonella mittels Immunoassay, **dadurch gekennzeichnet, daß** darin ein Antikörper nach Anspruch 3 enthalten ist.

## Claims

1. Peptide, selected from the protein PagC, **characterized by** a sequence according to one of the formulae Ia to Ie (Seq. I.D. No.: 1 - Seq. I.D. No.: 5).

Glu Pro Glu Gly Ile His Tyr His Asp Lys Phe Glu Val    Ia
                                        (Seq.I.D.No.: 1)

Ala Thr Phe Lys Glu His Ser Thr Gln Asp Gly Asp Ser    Ib
(Seq.I.D.No.: 2)

Gly Asp Arg Ala Ser Gly Ser Val Glu Pro Glu Gly  Ic
(Seq.I.D.No.: 3)

Gln Ser Lys Val Gln Asp Phe Lys Asn Ile Arg      Id
                                        (Seq.I.D.No.: 4)

Glu His Ser Thr Gln Asp Gly Asp Ser Phe Ser Asn Lys    Ie
                                        (Seq.I.D.No.: 5)

2. Use of a peptide according to Claim 1 for the production of immunogenic conjugates.

3. Antibody which is specifically directed against an epitope which is formed by a peptide according to Claim 1.

4. Use of an antibody according to Claim 3 for the detection of bacteria of the genus Salmonella.

5. Method for the detection of bacteria of the genus Salmonella by means of an immune reaction, **characterized in that** an antibody according to Claim 3 is used.

6. Test composition for the detection of bacteria of the genus Salmonella by means of immunoassay, **characterized in that** an antibody according to Claim 3 is contained therein.

## Revendications

1. Peptide choisi dans la protéine PagC, **caractérisé par** une séquence selon l'une des formules Ia à Ie (SEQ ID n° 1 - SEQ ID n° 5),

Glu Pro Glu Gly Ile His Tyr His Asp Lys Phe Glu Val Ia
(SEQ ID n° 1)

Ala Thr Phe Lys Glu His Ser Thr Gln Asp Gly Asp Ser Ib
(SEQ ID n° 2)

Gly Asp Arg Ala Ser Gly Ser Val Glu Pro Glu Gly Ic
(SEQ ID n° 3)

Gln Ser Lys Val Gln Asp Phe Lys Asn Ile Arg Id
(SEQ ID n° 4)

Glu His Ser Thr Gln Asp Gly Asp Ser Phe Ser Asn Lys Ie
(SEQ ID n° 5)

2. Utilisation d'un peptide selon la revendication 1, pour la préparation de conjugués immunogènes.

3. Anticorps dirigé spécifiquement contre un épitope qui est constitué d'un peptide selon la revendication 1.

4. Utilisation d'un anticorps selon la revendication 3, pour la détection de bactéries appartenant au genre *Salmonella.*

5. Procédé pour la détection de bactéries appartenant au genre *Salmonella,* au moyen d'une réaction immunologique, **caractérisé en ce qu'**on utilise un anticorps selon la revendication 3.

6. Ensemble d'essai pour la détection de bactéries appartenant au genre *Salmonella,* au moyen d'un dosage immunologique, **caractérisé en ce qu'**un anticorps selon la revendication 3 est contenu dans celui-ci.
